(19) Europäisches Patentamt / European Patent Office / Office européen des brevets

(11) **EP 4 218 827 A1**

(12) **EUROPEAN PATENT APPLICATION**
published in accordance with Art. 153(4) EPC

(43) Date of publication:
**02.08.2023 Bulletin 2023/31**

(21) Application number: **21872947.3**

(22) Date of filing: **24.09.2021**

(51) International Patent Classification (IPC):
**A61K 48/00** (2006.01)  **A61K 31/7088** (2006.01)
**A61K 47/54** (2017.01)  **A61K 47/64** (2017.01)
**A61P 25/28** (2006.01)

(52) Cooperative Patent Classification (CPC):
**A61K 31/7088; A61K 47/54; A61K 47/64;**
**A61K 48/00; A61P 25/28**

(86) International application number:
**PCT/KR2021/013049**

(87) International publication number:
**WO 2022/065919 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2020 KR 20200124531**

(71) Applicant: **Seasun Therapeutics**
**Daejeon 34015 (KR)**

(72) Inventors:
• **PARK, Min-Jung**
**Daejeon 35064 (KR)**
• **KIM, Hye Joo**
**Daejeon 34080 (KR)**
• **YU, Ji-Yeon**
**Cheongju-si, Chungcheongbuk-do 28193 (KR)**
• **PARK, Hee Kyung**
**Daejeon 34034 (KR)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **COMPOSITION FOR PREVENTING OR TREATING DEMENTIA, CONTAINING PEPTIDE NUCLEIC ACID COMPLEX AS ACTIVE INGREDIENT**

(57)   The present invention relates to a pharmaceutical composition for preventing or treating dementia, containing a nucleic acid complex in which a carrier peptide nucleic acid and a bioactive peptide nucleic acid having a sequence for binding to a NLRP3 gene are complementarily bound. The nucleic acid complex according to the present invention has a blood-brain barrier penetration ability and can effectively inhibit expression and phosphorylation of the protein of the NLRP3 gene and tau, which a lower-level gene, and thus is useful in the prevention or treatment of dementia.

Fig. 4

# Description

## Technical Field

The present invention relates to a pharmaceutical composition for preventing or treating dementia comprising a nucleic acid complex as an active ingredient and more particularly, to a pharmaceutical composition for preventing or treating dementia comprising a nucleic acid complex in which a bioactive peptide nucleic acid having a sequence capable of binding to an NLRP3 gene and a carrier peptide nucleic acid complementarily bound to each other.

## Background Art

Dementia is a pathological phenomenon that is distinguished from normal aging and known causes of such dementia include Alzheimer's disease, vascular dementia, Parkinson's disease, Lewy body dementia, Huntington's disease, Creutzfeldt-Jacob disease, Pick's disease and the like.

Among these causative diseases, Alzheimer's disease has the highest incidence rate and accounts for 50-70% of all dementia patients. Alzheimer's disease is classified into sporadic Alzheimer's disease and familial Alzheimer's disease depending on the cause thereof, and it is known that 80 to 90% of patients are sporadic with unknown causes, and most thereof occur after the age of 65 (Selkoe, 2001 Physiol Rev 81:741-66). Familial Alzheimer's disease accounts for less than 20% of the total Alzheimer's disease, mutations in genes such as amyloid precursor proteins (hereinafter referred to as APPs) and presenilin (PS) type 1 and 2 are the main causes of familial Alzheimer's disease, and familial Alzheimer's disease occurs even under the age of 65. Alzheimer's disease has pathological features in which senile plaques and neurofibrillary tangles accumulate outside and inside nerve cells, respectively. Senile plaques are spherical in shape, are generally found in the limbic system or neocortex and have a structure in which peptides called "beta-amyloids" ($\beta$-amyloid; hereinafter referred to as "A$\beta$") bind to one another and deposit to form a nucleus, and degenerated nerve fiber axons, dendrites, activated microglia and astrocytes gather around the nucleus. Neurofibrillary tangles are substances in nerve cells that are formed after proteins called "tau" are abnormally hyperphosphorylated and aggregate together, and are often found in many brain tissues of patients with dementia such as Alzheimer's disease. Tau protein is a type of microtubule-binding protein that regulates exon transport and stabilizes microtubules. However, hyperphosphorylation of tau protein abnormally increases in the Alzheimer's disease model, and the hyperphosphorylated tau protein aggregates in the form of PHFs (paired helical filaments). As a result, it is known that tau protein loses its function as a microtubule-binding protein and causes Alzheimer's disease.

The NLRP3 inflammasome in the brain is an immune complex found in the cytoplasm of microglia, which is an immune cell. Normally, NLRP3 inflammasome is present in an inactive state, whereas, when danger signals such as PAMPs (pathogen-associated molecular patterns) or DAMPs (damage-associated molecular patterns) are recognized, NLRP3 is activated and various kinds of proteins such as pro-caspase-1, ASC, and pro-IL-1$\beta$ gather to form the NLRP3 inflammasome as an oligomer.

It has recently been reported that the NLRP3 inflammasome, an innate immune factor, increases hyperphosphorylated tau protein through the downstream signaling pathway of amyloid beta pathology in the brain of Alzheimer's disease, which shows that amyloid beta plaques activate the NLRP3 inflammasome and directly correlate with tau protein. These recent studies show that when amyloid beta is activated, the NLRP3 inflammasome is activated and hyperphosphorylated tau protein is increased due to an increase in the inflammatory response of microglia (Ising, C. et al., Nature 575, 669-673, 2019).

Drugs currently on the market include Cognex® (tacrine), Aricept® (donepezil), Exelon® (rivastigmine), and Reminyl® (galantamine) as acetylcholine esterase activity inhibitors that maintain the concentration of acetylcholine as a neurotransmitter related to memory, to improve memory and provide short-term memory alleviation effect, and memantine (Ebixa®) as an NMDA receptor antagonist that inhibits Ca$^{2+}$-induced neuronal cell death. In addition, blood circulation enhancers such as nicergoline, L-carnitine, and ginkgo leaf extracts that have been identified to be indirectly effective for vascular dementia are being used.

Disadvantageously, most conventional dementia drugs are cholinergic and thus are inapplicable to administration in combination with other cholinergic drugs, neutralize the effect of anticholinergic drugs, and have a vagal hyperactivity effect on heart rate in the cardiovascular system. In addition, conventional dementia drugs disadvantageously should be used with caution in patients with a history of severe asthma or obstructive pulmonary disease. In addition, a great number of drugs developed as therapeutic agents for brain diseases have a problem in that they do not cross the blood-brain barrier well. The mechanism of crossing the blood-brain barrier has not yet been elucidated, even if a disorder occurs in the central nervous system, it is impossible to deliver drugs to the target area of the central nervous system, and effective treatment methods have not yet been developed.

For this reason, it is most important to find a drug that can effectively treat and prevent dementia in a different manner from conventional therapeutic agents and has low toxicity in order to treat dementia.

Meanwhile, unlike traditional drugs, nucleic acid drugs inhibit the expression of target-specific messenger RNA (mRNA) and thus research is ongoing into the use of nucleic acid drugs in areas in which treatment with conventional drugs that target proteins is impossible (Kole R. et al., Nature Rev. Drug Discov. 11;125, 2012, Wilson C. et al., Curr. Opin. Chem. Bio. 2006; 10:607, 2006). Although various clinical trials using nucleic acids are in progress due to performance and advantages thereof as drugs and the increasing use of nucleic acid-based therapeutics, the use of carriers for intracellular introduction or blood-brain barrier penetration is extremely limited.

Thus, recently, in order to promote safer and more effective use of drugs, not only formulations suitable for a drug but also methods of efficiently delivering drugs to target sites in the body are receiving attention. In particular, there is demand for practical application of a drug delivery system (DDS) that efficiently transports a drug in a required amount to a required place when needed. The development of a carrier that is able to deliver a drug through the blood-brain barrier is also actively underway.

In this regard, the present inventors have found that a nucleic acid complex in which a bioactive nucleic acid complementarily binds to a carrier peptide nucleic acid modified to have an overall positive charge has surprisingly improved cell permeability, and this enables the expression of the target gene to be regulated very efficiently, and filed a patent application on a novel construct with low cytotoxicity and improved cell permeability and ability to regulate gene expression of bioactive nucleic acids (PCT/KR2017/008636). In addition, the present inventors have continuously conducted research on the function of the construct and developed a novel construct having the ability to cross the blood-brain barrier with excellent efficiency (KR 10-2019-0128465).

Accordingly, as a result of intensive efforts to apply nucleic acid complexes having excellent blood-brain barrier permeability to the treatment of dementia, the present inventors have found that a nucleic acid complex in which a bioactive peptide nucleic acid targeting an NLRP3 gene and a carrier peptide nucleic acid complementarily bound to each other is highly effective in preventing or treating dementia and thus completed the present invention.

## Summary of the Invention

It is an object of the present invention to provide a pharmaceutical composition for preventing or treating dementia comprising a nucleic acid complex as an active ingredient.

In order to accomplish the above object, the present invention provides a pharmaceutical composition for preventing, ameliorating or treating Alzheimer's dementia comprising a cell-permeable nucleic acid complex in which a bioactive nucleic acid targeting an NLRP3 gene and a carrier peptide nucleic acid complementarily bound to each other, as an active ingredient.

In addition, the present invention provides a method of preventing or treating dementia comprising administering the cell-permeable nucleic acid complex.

In addition, the present invention provides the use of the cell-permeable nucleic acid complex for the prevention or treatment of dementia.

In addition, the present invention provides the use of the cell-permeable nucleic acid complex for the manufacture of a medicament for preventing or treating dementia.

## Brief Description of Drawings

FIG. 1 shows the results of Western blot assay of the expression of dementia-related genes (NLRP3 and a downstream gene thereof) after treatment of mouse microglia (BV-2) with the peptide nucleic acid complex of the present invention.

FIG. 2 shows the results of Western blot assay of the expression of dementia-related genes after treatment of primary microglia with the peptide-nucleic acid complex of the present invention.

FIG. 3 shows changes in the expression of NLRP3 and the downstream gene thereof (A) and changes in the expression of p-tau, the putative gene for Alzheimer's disease (B) in primary microglia having the immune response activated by treatment with LPS/ATP, based on the results of Western blot assay.

FIG. 4 shows changes in the expression of NLRP3 and the downstream gene thereof (A) and changes in the expression of p-tau, the putative gene for Alzheimer's disease (B) after treatment with the peptide-nucleic acid complex of the present invention to primary microglia treated with LPS/ATP, based on the results of the Western blot assay.

## Detailed Description and Preferred Embodiments of the Invention

Unless defined otherwise, all technical and scientific terms used herein have the same meanings as appreciated by those skilled in the field to which the present invention pertains. In general, the nomenclature used herein is well-known in the art and is ordinarily used.

The present invention is based on the finding that a nucleic acid complex in which a bioactive peptide nucleic acid and a carrier peptide nucleic acid complementarily bound to each other is capable of penetrating the blood-brain barrier, in particular, a nucleic acid complex in which a bioactive peptide nucleic acid targeting an NLRP3 gene and a carrier peptide nucleic acid complementarily bound to each other efficiently inhibits the NLRP3 gene and the protein expression and phosphorylation of the downstream gene thereof, tau. That is, the nucleic acid complex according to the present invention, in which a bioactive peptide nucleic acid having a sequence capable of binding to an NLRP3 gene and a carrier peptide nucleic acid complementarily bound to each other, may be used for the treatment of dementia.

Accordingly, in one aspect, the present invention is directed to a pharmaceutical composition for preventing or treating Alzheimer's dementia comprising a cell-permeable nucleic acid complex in which a bioactive peptide nucleic acid having a sequence capable of binding to an NLRP3 gene and a carrier peptide nucleic acid complementarily bound to each other, as an active ingredient.

In the present invention, the nucleic acid complex in which the bioactive peptide nucleic acid and the carrier peptide nucleic acid complementarily bound to each other may have a structure represented by the following Structural Formula (1):

$$\text{Structural Formula (1)}$$

$$[\ A \equiv C^{(+)}\ ]$$

Wherein Structural Formula (1),

A is a bioactive nucleic acid having a sequence capable of binding to a target gene or a target gene sequence,
C is a carrier peptide nucleic acid capable of binding to the bioactive nucleic acid, and
'$\equiv$' means complementary binding between the bioactive nucleic acid and the carrier peptide nucleic acid,
wherein the bioactive nucleic acid represented by A is a peptide nucleic acid which has an overall negative charge,
the carrier peptide nucleic acid represented by $C^{(+)}$ has an overall positive charge,
the nucleic acid complex of Structural Formula (1) represented by $A \equiv C^{(+)}$ has an overall positive charge, and
the carrier peptide nucleic acid has an overall positive charge because it includes one or more gamma- or alpha-backbone modified peptide nucleic acid monomers to impart the overall positive charge to the carrier peptide nucleic acid, and the gamma- or alpha-backbone modified peptide nucleic acid monomers include more monomers having negatively charged amino acids than monomers having negatively charged amino acids.

As used herein, the term "bioactive nucleic acid" refers to a nucleic acid having a complementary sequence capable of binding to a gene that is the target of expression inhibition, in particular, a nucleic acid having a complementary sequence capable of binding to the mRNA of the gene that is the target of expression inhibition, or a nucleic acid having a sequence that is the target of the expression promotion, means a nucleic acid involved in the regulation of gene expression, such as inhibiting or promoting the expression of the corresponding gene, or a nucleic acid having a sequence complementary to a gene that is the target of expression inhibition or promotion, or a nucleic acid having a sequence complementary to a single-stranded RNA sequence such as pre-mRNA, miRNA, and mRNA

In particular, the "bioactive peptide nucleic acid" used herein binds to a target gene and a nucleotide sequence comprising the same *in vitro* or *in vivo* and thus acts to activate or inhibit the inherent functions (transcript expression or protein expression) of the corresponding gene, or to regulate splicing of pre-mRNA (e.g., exon skipping), and the base sequence is a gene regulatory sequence, gene coding sequence or a splicing regulatory sequence. The gene regulatory region is a promoter, a transcriptional enhancer, a 5' untranslated region, a 3' untranslated region, a viral packaging sequence, and a selectable marker. The gene region may be an exon or an intron, and the gene region may be present within 10, 5, 3, or 1 kb or 500, 300, or 200 bp of the transcription initiation site of the gene, for example, upstream or downstream of the initiation site. In addition, the splicing regulatory region may comprise sequences related to exon skipping, cryptic splicing, pseudo-splice site activation, intron retention, and alternative splicing deregulation.

Therefore, the "bioactive nucleic acid" in the present invention is preferably an antisense peptide nucleic acid of NLRP3 (NLR family pyrin domain containing 3), which is a target gene for dementia diseases, and comprises more preferably any one of sequences represented by SEQ ID NOS: 1 to 3, but is not limited thereto.

As used herein, the term "carrier peptide nucleic acid" refers to a nucleic acid, the bases of which are partly or entirely complementarily bound to the bioactive nucleic acid, to impart functionality thereto, and the carrier peptide nucleic acid used herein may be a peptide nucleic acid (PNA) or a modified nucleic acid similar thereto, and is preferably a peptide nucleic acid, but is not limited thereto.

In particular, the carrier peptide nucleic acid preferably comprises a sequence selected from the group consisting of sequences represented by SEQ ID NOS: 5 to 14, but is not limited thereto.

As used herein, the nucleic acid complex may be in a de-salted form.

As used herein, the nucleic acid complex is capable of allowing the bioactive substance to enter the body, ultimately cells and specifically is capable of delivering the bioactive substance into the body through the blood-brain barrier.

Accordingly, in the present invention, the nucleic acid complex may be capable of penetrating the blood-brain barrier.

In the present invention, each of the bioactive nucleic acid and the carrier peptide nucleic acid comprises 2 to 50, preferably 5 to 30, more preferably 10 to 25, most preferably 15 to 17 nucleic acid monomers.

In the present invention, the nucleic acid complex comprises a bioactive nucleic acid having sequences represented by SEQ ID NOS: 1 to 3, and a carrier peptide nucleic acid comprising a sequence represented by any one selected from the group consisting of SEQ ID NOS: 5 to 14, but is not limited thereto.

The composition of the present invention comprises, as an active ingredient, a nucleic acid complex comprising a bioactive nucleic acid having a sequence represented by SEQ ID NO: 1 and a carrier peptide nucleic acid having the sequence represented by SEQ ID NO: 14, a nucleic acid complex comprising a bioactive nucleic acid having a sequence represented by SEQ ID NO: 3 and a carrier peptide nucleic acid having the sequence represented by SEQ ID NO: 10, or a nucleic acid complex comprising a bioactive nucleic acid having a sequence represented by SEQ ID NO: 2 and a carrier peptide nucleic acid having the sequence represented by SEQ ID NO: 7, but is not limited thereto.

In the present invention, the bioactive nucleic acid or the carrier peptide nucleic acid may be further bound with a substance for facilitating endosomal escape to the 5'-end or the 3'-end of each nucleic acid. That is, the bioactive nucleic acid or the carrier peptide nucleic acid may further comprise a substance for facilitating endosomal escape of the bioactive nucleic acid and the carrier peptide nucleic acid and thus have the structure represented by the following Structural Formula (2).

Structural Formula (2)

$$[ \ mA \ \equiv \ mC^{(+)} \ ]$$

Wherein Structural Formula (2),

"m" means a substance for facilitating endosomal escape of the bioactive nucleic acid and the carrier peptide nucleic acid.

In the present invention, the "substance for facilitating endosomal escape" may facilitate escape of the bioactive nucleic acid from the endosomes by increasing the osmotic pressure in the endosomes or destabilizing the membrane of the endosomes, which means that the "substance for facilitating endosomal escape" enables the bioactive nucleic acid to move more efficiently and rapidly to the nucleus or cytoplasm and to meet and function with the target gene (D. W. Pack, A. S. Hoffman, S. Pun, P. S. Stayton, "Design and development of polymers for gene delivery," Nat. Rev. Drug. Discov., 4, 581-593 (2005)).

In the present invention, the substance for facilitating endosomal escape is at least one selected from the group consisting of peptides, lipid nanoparticles, polyplex nanoparticles, polymer nanospheres, inorganic nanoparticles, cationic lipid-based nanoparticles, cationic polymers, and pH-sensitive polymers.

In the present invention, as the substance for facilitating endosomal escape, a peptide having a sequence of GLFDIIKKIAESF (SEQ ID NO: 15) may be bound to the bioactive nucleic acid via a linker, and histidine (10) may be bound to the carrier peptide nucleic acid via a linker, but the present invention is not limited thereto.

In the present invention, the lipid nanoparticles may be selected from the group consisting of lipids, phospholipids, cetyl palmitate, poloxamer 18, Tween 85, tristearin glyceride, and Tween 80.

In the present invention, the polyplex nanoparticles may be poly(amidoamine) or polyethylenimine (PEI).

In the present invention, the polymer nanospheres may be selected from the group consisting of polycaprolactone, poly(lactide-co-glycolide), polylactide, polyglycolide, poly(d,l-lactide), chitosan, and PLGA-polyethylene glycol.

In the present invention, the inorganic nanoparticles may be selected from the group consisting of $Fe_2O_3$, $Fe_3O_4$, $WO_3$ and $WO_{2.9}$.

In the present invention, the cationic lipid-based nanoparticles may be selected from the group consisting of 1-(aminoethyl)iminobis[N-(oleicylcysteinyl-1-aminoethyl)propionamide], an N-alkylated derivative of PTA and 3,5-bis(dodecyloxy)benzamidine.

In the present invention, the cationic polymer may be selected from the group consisting of vinylpyrrolidone-N,N-dimethylaminoethyl methacrylate acid copolymer diethyl sulphate, polyisobutylene, and poly(N-vinylcarbazole).

In the present invention, the pH-sensitive polymers may be selected from the group consisting of polyacids, poly(acrylic acid), poly (methacrylic acid) and hydrolyzed polyacrylamide.

The bioactive nucleic acid may comprise a natural nucleic acid base and/or a modified nucleic acid monomer. The carrier peptide nucleic acid may have a sequence partially or entirely complementary to the base sequence of the bioactive nucleic acid.

In particular, the carrier peptide nucleic acid may comprise one or more universal bases and all of the carrier peptide nucleic acids may comprise universal bases.

In the present invention, the bioactive nucleic acid is selected from the group consisting of DNA, RNA, or modified nucleic acids including peptide nucleic acid (PNA), phosphorodiamidate morpholino oligonucleotide (PMO), locked nucleic acid (LNA), glycol nucleic acid (GNA), and threose nucleic acid, antisense oligonucleotide, aptamers, small interfering RNA (siRNA), short hairpin RNA (shRNA), ribozyme, and DNAzyme. Preferably, the bioactive nucleic acid is selected from the group consisting of DNA, RNA, or modified nucleic acids including peptide nucleic acid (PNA), phosphorodiamidate morpholino oligonucleotide (PMO), locked nucleic acid (LNA), glycol nucleic acid (GNA), and threose nucleic acid (TNA).

In the present invention, when the monomer used for the bioactive nucleic acid is PNA, the monomer is called a "bioactive peptide nucleic acid". When other monomers are also used, they are called in the same manner as above.

In the present invention, the bioactive nucleic acid and the carrier peptide nucleic acid may further comprise at least one functional group selected from the group consisting of phosphodiester, 2'-0-methyl, 2'-methoxy-ethyl, phosphoramidate, methylphosphonate, and phosphorothioate.

In the present invention, the carrier peptide nucleic acid may have a nucleotide sequence which is partially or entirely complementary to the bioactive nucleic acid. In particular, the carrier peptide nucleic acid may comprise at least one universal base, and the carrier peptide nucleic acid may be entirely composed of universal bases.

In the present invention, with regard to the electrical properties, each of the bioactive nucleic acid and the carrier peptide nucleic acid in the nucleic acid complex may have an overall positive charge (positive), an overall negative charge (negative), or no charge (neutral).

The term "overall" in the expression of the electrical properties means overall electrical properties of respective charges of the bioactive nucleic acids or carrier peptide nucleic acids as viewed from the outside, not the electrical properties of individual bases. For example, even though some monomers in the bioactive nucleic acid are positive, when the number of negatively charged monomers is greater than the number of positively charged monomers, the bioactive nucleic acid is negatively charged in terms of the "overall" electrical properties. When the number of positively charged bases and/or backbones is greater than the number of negatively charged bases and/or backbones, even though some bases and/or backbone constituents in the carrier peptide nucleic acid are negatively charged, the carrier peptide nucleic acid is considered to be positively charged in terms of the "overall" electrical properties thereof.

Accordingly, the nucleic acid complex of the present invention may be considered to have an overall positive charge. In the nucleic acid complex, preferably, the bioactive nucleic acid has an overall negative charge or is neutral in terms of overall electrical properties, and the carrier peptide nucleic acid has an overall positive charge in terms of overall electrical properties, but is not limited thereto.

In the present invention, a modified peptide nucleic acid monomer may be used to impart electrical properties to the bioactive nucleic acid and the carrier peptide nucleic acid, and the modified peptide nucleic acid monomer comprises, as a positively charged carrier peptide nucleic acid, at least one positively charged nucleic acid selected from the group consisting of lysine (Lys, K), arginine (Arg, R), histidine (His, H), diamino butyric acid (DAB), ornithine (Orn), and amino acid analogs, and comprises, as a negatively charged carrier peptide nucleic acid, glutamic acid (Glu, E), which is a negatively charged amino acid, or an amino acid analogue, which is a negatively charged amino acid.

In the present invention, the carrier peptide nucleic acid may comprise at least one gamma- or alpha-backbone-modified peptide nucleic acid monomer in order for the carrier peptide nucleic acid to have an overall positive charge.

The gamma- or alpha-backbone-modified peptide nucleic acid monomer comprises, in the backbone thereof, at least one positively charged amino acid selected from the group consisting of lysine (Lys, K), arginine (Arg, R), histidine (His, H), diamino butyric acid, ornithine (Orn), and amino acid analogs in order for the carrier peptide nucleic acid to have an overall positive charge.

In the present invention, the peptide nucleic acid monomer having a modified nucleobase, in addition to the backbone modification, for modification of the peptide nucleic acid monomer so as to impart an electrical charge thereto may be used. Preferably, an amine, triazole or imidazole moiety may be comprised in the nucleobase to impart a positive charge thereto, or carboxylic acid may be comprised in the base to impart a negative charge thereto.

In the present invention, the modified peptide nucleic acid monomer of the carrier peptide nucleic acid may further comprise a negative charge in the backbone or nucleobase. However, preferably, the modified peptide nucleic acid monomer comprises more positively charged monomers than negatively charged monomers, such that the carrier peptide nucleic acid is positively charged.

In the nucleic acid complex according to the present invention, at least one material selected from the group consisting of a hydrophobic moiety, a hydrophilic moiety, a target-antigen-specific antibody, an aptamer, and a fluorescent/luminescent marker is bound to the bioactive nucleic acid and/or the carrier peptide nucleic acid. Preferably, at least one material selected from the group consisting of a hydrophobic moiety, a hydrophilic moiety, a target-antigen-specific antibody, an aptamer and a fluorescent/luminescent marker for imaging may be bound to the carrier peptide nucleic acid.

In the present invention, the binding of at least one material selected from the group consisting of a hydrophobic moiety, a hydrophilic moiety, a target-antigen-specific antibody, an aptamer, a quencher, a fluorescent marker and a luminescent marker to the bioactive nucleic acid and/or the carrier peptide nucleic acid may be a simple covalent bond or a covalent bond mediated by a linker, but is not limited thereto. Preferably, cell permeation, solubility, stability, transportation and imaging-related substances (e.g., hydrophobic residues and the like) bound to the nucleic acid carrier exist independently of the bioactive nucleic acid that regulates the expression of the target gene.

In the present invention, as described above, the complementary binding of the bioactive nucleic acid and the carrier peptide nucleic acid is generally antiparallel binding or parallel binding. Complementary binding forms a structure that is separated in the presence of the target sequence of the bioactive nucleic acid (a sequence complementary to the bioactive nucleic acid).

The antiparallel binding and parallel binding are determined depending on the 5'-direction and the 3'-direction in the binding mode of DNA-DNA or DNA-PNA. Antiparallel binding is a general binding mode of DNA-DNA or DNA-PNA. For example, in the nucleic acid complex according to the present invention, the bioactive nucleic acid in a 5' to 3' direction is bound to the carrier peptide nucleic acid in a 3' to 5' direction. Parallel binding has slightly lower binding force than antiparallel binding, and the bioactive nucleic acid and the carrier peptide nucleic acid are bound to each other in the same direction, that is, the 5' to 3' direction or the 3' to 5' direction.

In the nucleic acid complex according to the present invention, preferably, the binding force between the bioactive nucleic acid and the carrier peptide nucleic acid is smaller than the binding force between the bioactive nucleic acid and the target gene targeted by the bioactive nucleic acid, particularly the mRNA of the target gene. The bonding force is determined by the melting temperature (Tm).

Examples of specific methods for lowering the binding force (melting temperature, Tm) between the bioactive nucleic acid and the carrier peptide nucleic acid, compared to the binding force between the bioactive nucleic acid and the target gene targeted by the bioactive nucleic acid, particularly the mRNA of the target gene, include parallel binding or partial specific binding between the bioactive nucleic acid and the carrier peptide nucleic acid, but are not limited thereto.

As another example, the carrier peptide nucleic acid comprises at least one peptide nucleic acid base selected from the group consisting of a linker, a universal base, and a peptide nucleic acid base comprising a base that is not complementary to a corresponding base of the bioactive nucleic acid, but is not limited thereto.

In the present invention, the universal base is a base that is non-selectively bound to a natural base such as adenine, guanine, cytosine, thymine or uracil, and has a binding force lower than the complementary binding force, and includes at least one selected from the group consisting of inosine PNA, indole PNA, nitroindole PNA, and abasic PNA, and is preferably inosine PNA.

The present invention provides a combination of the binding mode and electrical properties of nucleic acids for controlling the function of the nucleic acid complex, and controls the particle size and the action time using the combination of the binding mode and electrical properties of the nucleic acids, and improves cell permeability, solubility and specificity.

In the present invention, the time point at which the bioactive peptide nucleic acid binds to the target sequence (such as the time point at which the bioactive nucleic acid is substituted with the target sequence, and the time point at which target-specific release and binding occur) may be controlled in the presence of the target gene through control of the binding force between the bioactive peptide nucleic acid and the carrier peptide nucleic acid.

In the nucleic acid complex according to the present invention, the control of the time point of substitution (strand displacement) of the bioactive nucleic acid with the target gene and the time point of target-specific release and binding is made possible by the presence, number, and position of non-specific bases, universal bases and linkers of carrier nucleic acids for non-specific binding of the complex. The control is possible due to the combination of the factors described above and parallel or antiparallel binding, which is the complementary binding mode of the peptide complex.

As used herein, the term "blood-brain barrier" is used interchangeably with BBB and refers to a permeable barrier present within the blood because the blood circulates through brain tissue that closely regulates and strictly restricts the exchange between blood and brain tissue. Components of the blood-brain barrier include endothelial cells that form the deepest lining of all blood vessels, dense junctions between adjacent endothelial cells that are structurally correlated with the BBB, the basement membrane of endothelial cells, and an extended foot process of adjacent astrocytes covering almost all of the exposed outer surface of blood vessels. The BBB prevents most substances in the blood, including most large molecules therein, such as Ig, antibodies, complements, albumin, and drugs and small molecules, from entering brain tissue.

As used herein, the terms "disease" and "disorder" are used interchangeably and refers to any change in the state of the body or in some organs which impede and/or disrupt the performance of a function, cause symptoms such as discomfort and dysfunction, or lead to the death of people with a disease or the death of people who come into contact therewith.

In the present invention, the disease is preferably dementia, and is more preferably selected from the group consisting of Alzheimer's disease, vascular dementia, dementia caused by Parkinson's disease, Lewy body dementia, dementia caused by Huntington's disease, dementia caused by Creutzfeldt-Jacob disease, and dementia caused by Pick's disease, but is not limited thereto.

In the present invention, the term "therapeutic composition" may be used interchangeably with "pharmaceutical composition", and refers to a composition that comprises a nucleic acid complex comprising the bioactive nucleic acid and the carrier peptide nucleic acid bound to the nucleic acid according to the present invention as an active ingredient, and may further comprise a therapeutic drug for treating a desired disease, bound to the nucleic acid complex.

The therapeutic composition of the present invention may be formulated in a form that can be delivered into the blood-brain barrier according to standard pharmaceutical practice. In addition to the active ingredient, these formulations may comprise additives such as carriers, excipients, adjuvants or diluents suitable for pharmaceutically acceptable formulations.

The term "physiologically acceptable" refers to a property that does not impair the biological activity and physical properties of a compound.

The term "carrier" is defined as a compound that facilitates the transport of the nucleic acid complex into cells or tissues. For example, dimethylsulfoxide (DMSO) is a commonly used carrier that facilitates the incorporation of many organic compounds into cells or tissues of an organism.

The term "diluent" is defined as a compound that stabilizes a biologically active form of a target compound and is diluted in water that dissolves the target compound. Salts dissolved in buffer solutions are used as diluents in the art. A commonly used buffer solution is phosphate-buffered saline because it mimics the salinity of human bodily fluids. Because buffer salts can control the pH of a solution at low concentrations, buffer diluents rarely alter the biological activity of compounds.

The substance comprising the nucleic acid complex in the present invention may be administered to a patient alone or as a pharmaceutical composition mixed with other active ingredients, or with suitable carriers or excipients, that is, in combination therapy.

The pharmaceutical composition suitable for use in the present invention comprises compositions comprising the active ingredients in an amount effective to achieve the intended purpose thereof. More specifically, a therapeutically effective amount means an amount of a compound effective to lengthen the survival of the subject to be treated, or to prevent, alleviate or relieve the symptoms of diseases. The determination of the therapeutically effective amount is possible by those skilled in the art, particularly in consideration of the detailed description provided herein.

The term "prevention" as used herein means any action of preventing the onset of a disease or inhibiting the progression thereof by administration of the therapeutic composition comprising the nucleic acid complex.

The term "alleviation" as used herein refers to any action of at least reducing the degree of parameters related to the condition to be treated, for example, the severity of symptoms, by administration of the therapeutic composition comprising the nucleic acid complex.

In addition, the term "treatment" as used herein refers to any action in which symptoms of a disease are alleviated or eliminated by administration of the therapeutic composition comprising the nucleic acid complex.

The composition comprising the nucleic acid complex according to the present invention may be applied to the skin in a pharmaceutically effective amount to treat dementia or to inhibit (or alleviate) the symptoms of dementia. The pharmaceutically effective amount may vary depending on various factors such as the type of dementia, the age and weight of the patient, the characteristics and extent of symptoms, the type of current therapy, the number of treatments that are performed, and the application form and route, and can be easily determined by experts in the field. The composition of the present invention may be applied simultaneously or sequentially in combination with the pharmacological or physiological components described above, and may be applied sequentially or simultaneously in combination with additional conventional therapeutic agents. Administration may be performed in one or multiple applications.

As used herein, the term "subject" refers to a mammal, preferably a human, that suffers from or is at risk of a condition or disease that can be alleviated, suppressed or treated by administering the nucleic acid complex according to the present invention thereto.

In addition, the amount of the compound of the present invention that is administered to the human body may vary depending on the age, weight and gender of the patient, the administration form, the health status, and the severity of disease, and is generally 0.001 to 1,000 mg/day, preferably 0.01 to 500 mg/day, based on an adult patient weighing 70 kg, and may be administered once a day or in multiple doses (in a portionwise manner) several times a day at regular time intervals according to the prescription of doctors or pharmacists.

The toxicity and therapeutic efficiency of the compositions comprising the nucleic acid complex described herein are, for example, estimated through standard pharmaceutical procedures in cell culture or laboratory animals to determine the LD50 (lethal dose for 50% of the population), ED50 (dose providing a therapeutic effect on 50% of the population) and IC50 (dose providing therapeutic and inhibitory effects on 50% of the population). The ratio of toxicity to therapeutic effect for a dose is called the therapeutic index, and may be expressed as the ratio of LD50 to ED50 (or IC50). Compounds having a high therapeutic index are preferred. The data obtained from these cell culture assays may be used to estimate the range of dose for human applications. The amount (dosage) of such a compound that is administered is preferably within a range of a circulating concentration including ED50 (or IC50) with little or no toxicity.

As used herein, the term "administration" refers to an act of introducing the pharmaceutical composition of the present invention into a subject by any suitable method, and the administration may be performed through any of various routes, either oral or parenteral, as long as it enables the composition to reach the target tissue.

The pharmaceutical composition of the present invention may be administered in a pharmaceutically effective amount. The term "pharmaceutically effective amount" used herein means a sufficient amount used to treat or prevent a disease at a reasonable benefit/risk ratio applicable to medical treatment or prevention. The effective amount is determined depending on factors including the severity of the disease, the activity of the drug, the age, weight, health and gender of the patient, the sensitivity of the patient to the drug, the time of administration, the route of administration, and the rate of excretion and treatment period of the composition of the present invention used, drugs used in combination with or concurrently with the composition of the present invention, and other factors well known in the pharmaceutical field.

The pharmaceutical composition of the present invention may be administered as a single therapeutic agent or in combination with other therapeutic agents, either sequentially or simultaneously. The pharmaceutical composition of the present invention may be administered in single or multiple doses. Taking into consideration these factors, it is important to administer the composition in the minimum amount sufficient to achieve maximum efficacy without side effects.

In addition, the dosage (administered amount) of the pharmaceutical composition according to the present invention may be determined by those skilled in the art in consideration of the purpose of use, the severity of the disease, the patient's age, weight, gender, and history, or the substances used as active ingredients. For example, the pharmaceutical composition may be administered to an adult in a daily dose of 10 mg/kg to 100 mg/kg, more preferably 10 mg/kg to 30 mg/kg. The frequency of administration of the composition of the present invention is not particularly limited, and the composition may be administered one to three times a day, or may be divided into multiple doses and administered throughout the day.

In another aspect, the present invention is directed to a method of preventing or treating dementia comprising administering a nucleic acid complex in which a bioactive peptide nucleic acid having a sequence capable of binding to an NLRP3 gene and a carrier peptide nucleic acid complementarily bound to each other, and/or a composition comprising the nucleic acid complex, to a subject.

In another aspect, the present invention is directed to the use of a nucleic acid complex in which a bioactive peptide nucleic acid having a sequence capable of binding to an NLRP3 gene and a carrier peptide nucleic acid complementarily bound to each other, and/or a composition comprising the nucleic acid complex for the manufacture of a medicament for preventing or treating dementia.

In another aspect, the present invention is directed to the use of a nucleic acid complex in which a bioactive peptide nucleic acid having a sequence capable of binding to an NLRP3 gene and a carrier peptide nucleic acid complementarily bound to each other, and/or a composition comprising the nucleic acid complex for the prevention or treatment of dementia.

### [Example]

Hereinafter, the present invention will be described in more detail with reference to the following examples. However, it will be obvious to those skilled in the art that the following examples are provided only for illustration of the present invention and should not be construed as limiting the scope of the present invention based on the subject matter of the present invention.

### Example 1: Bioactive peptide nucleic acid and carrier peptide nucleic acid, and preparation of complex using the same

NLRP3 was used as a target gene to verify the effect on Alzheimer's dementia of the nucleic acid complex of the present invention. An antisense peptide nucleic acid (antisense PNA) was used as the bioactive nucleic acid for NLRP3 to determine the therapeutic effect on Alzheimer's dementia.

The bioactive nucleic acid (antisense PNA) used to determine the therapeutic effect on Alzheimer's dementia has sequences represented by SEQ ID NOS: 1 to 3 and the bioactive nucleic acid (antisense PNA) used as a control of the present invention has a sequence represented by SEQ ID NO: 4.

A peptide for facilitating the endosomal escape GLFDIIKKIAESF (SEQ ID NO: 15), was linked to the 5' end of the peptide-nucleic-acid-based bioactive nucleic acids used in this example which are represented by SEQ ID NOS: 2 to 4, and the sequence represented by SEQ ID NO: 1 was synthesized without the peptide for facilitating the endosomal escape. All carrier peptide nucleic acids used in this example, excluding the sequence represented by SEQ ID NO: 14 were linked the peptides for facilitating the endosomal escape to the 5' or 3' end thereof, and have sequences represented by SEQ ID NOS: 5 to 13. Base sequences, monomer modifications and structures are shown in Table 1 below. All peptide nucleic acids used in the present invention were synthesized by HPLC purification at Panagene (Korea) (Table 1).

[Table 1]

| Sequences of bioactive nucleic acids and carrier peptide nucleic acids to verify therapeutic effect for Alzheimer's disease | | | | |
| --- | --- | --- | --- | --- |
| Item | SEQ ID NO: | Endosome escape peptide | Base sequence | Monomer modification |
| Bioactive nucleic acid | SEQ ID NO: 1 | - | 5'-ACG$^{(-)}$TGC$^{(+)}$ATT$^{(-)}$AT$^{(+)}$CT$^{(-)}$GA-O-K-3' | -+-+- |
| | SEQ ID NO: 2 | GLFDIIKKI AESF | 5'-GLFDIIKKIAESF-O-TCG$^{(-)}$AT$^{(+)}$CAT$^{(-)}$TA$^{(+)}$GCG$^{(-)}$TG-O-K-3' | -+-+- |
| | SEQ ID NO: 3 | GLFDIIKKI AESF | 5'-GLFDIIKKIAESF-O-ACG$^{(-)}$TGC$^{(+)}$ATT$^{(-)}$AT$^{(+)}$CT$^{(-)}$GA-O-K-3' | -+-+- |
| | SEQ ID NO: 4 (Control) | GLFDIIKKI AESF | 5'-GLFDIIKKIAESF-O-AC$^{(-)}$CT$^{(+)}$CTA$^{(-)}$CGC$^{(+)}$AAT$^{(-)}$CC-O-K-3' | -+-+- |

(continued)

| Sequences of bioactive nucleic acids and carrier peptide nucleic acids to verify therapeutic effect for Alzheimer's disease | | | | |
|---|---|---|---|---|
| Item | SEQ ID NO: | Endosome escape peptide | Base sequence | Monomer modification |
| Carrier peptide nucleic acid | SEQ ID NO: 5 | Histidine(10) | 5'-Histidine(10)-O-TGGA$^{(+)}$GATG$^{(+)}$CGTT$^{(+)}$AGG-O-K-3' | +++ |
| | SEQ ID NO: 6 | Histidine(10) | 5'-Histidine(10)-O-AGC$^{(+)}$TAGTA$^{(+)}$ATCGC$^{(+)}$ACO-K-3' | +++ |
| | SEQ ID NO: 7 | Histidine(10) | 5'-K-O-CA$^{(+)}$CGCTA$^{(+)}$ATGAT$^{(+)}$CGA-O-Histidine(10)-3' | +++ |
| | SEQ ID NO: 8 | Histidine(10) | 5'-Histidine(10)-O-CG$^{(+)}$CA$^{(+)}$C-O-K-3' | ++ |
| | SEQ ID NO: 9 | Histidine(10) | 5'-K-O-CA$^{(+)}$CG$^{(+)}$C-O-Histidine(10)-3' | ++ |
| | SEQ ID NO: 10 | Histidine(10) | 5'-Histidine(10)-O-TGC$^{(+)}$ACGTAA$^{(+)}$TAGA$^{(+)}$CT-K-3' | +++ |
| | SEQ ID NO: 11 | Histidine(10) | 5'-K-O-TC$^{(+)}$AGAT$^{(+)}$AATGCA$^{(+)}$CGT-O-Histidine(10)-3' | +++ |

(continued)

| Item | SEQ ID NO: | Endosome escape peptide | Base sequence | Monomer modification |
|------|-----------|------------------------|---------------|---------------------|
| Sequences of bioactive nucleic acids and carrier peptide nucleic acids to verify therapeutic effect for Alzheimer's disease | | | | |
| | SEQ ID NO: 12 | Histidine(10) | 5'-Histidine(10)-O-A$^{(+)}$GA$^{(+)}$CT-O-K-3' | ++ |
| | SEQ ID NO: 13 | Histidine(10) | 5'-K-O-TC$^{(+)}$AG$^{(+)}$AC-O-Histidine(10)-3' | ++ |
| | SEQ ID NO: 14 | - | 5'-TGC$^{(+)}$ACGTAA$^{(+)}$TAGA$^{(+)}$CT-K-3' | +++ |

The above Table 1 shows sequence information of the bioactive nucleic acid and the carrier peptide nucleic acid to determine the effect as a therapeutic agent for Alzheimer's dementia targeting NLRP3, and sequence information of the bioactive nucleic acid and the carrier peptide nucleic acid used as a control group.

In order to impart electrical properties, modification of the monomer was performed to produce a modified peptide nucleic acid backbone is designed such that the peptide nucleic acid backbone comprises lysine (Lys, K$^{(+)}$) for a positive electrical charge and the modified peptide nucleic acid backbone comprises glutamic acid (Glu, E$^{(-)}$) for a negative electrical charge.

The combinations of respective bioactive nucleic acids and carrier peptide nucleic acids were hybridized in the presence of DMSO to produce a complex comprising the bioactive nucleic acid and the carrier peptide nucleic acid.

**Example 2: Analysis of therapeutic effect for Alzheimer's dementia using nucleic acid complexes**

The therapeutic effect on Alzheimer's dementia was analyzed using the nucleic acid complex comprising the bioactive peptide nucleic acid and the carrier peptide nucleic acid using NLRP3 as a target gene, prepared to have the structure of the following Table 2 according to Example 1.

[Table 2]

| Combination | Nucleic acid complex |
|-------------|---------------------|
| Combination of nucleic acid complexes for gene expression analysis in rat microglia | |
| 1 | SEQ ID NOS: 4 and 5 |
| 2 | SEQ ID NOS: 2 and 6 |
| 3 | SEQ ID NOS: 2 and 7 |
| 4 | SEQ ID NOS: 2 and 8 |
| 5 | SEQ ID NOS: 2 and 9 |
| 6 | SEQ ID NOS: 3 and 10 |
| 7 | SEQ ID NOS: 3 and 11 |
| 8 | SEQ ID NOS: 3 and 12 |
| 9 | SEQ ID NOS: 3 and 13 |

Example 2-1: Cell culture

Mouse microglia (BV-2) obtained from Elabscience (USA) were incubated at 37°C in the presence of 5% (v/v) $CO_2$ in DMEM culture medium (Dulbecco's Modified Eagle's Medium, Wellgene, Korea) supplemented with 10% (v/v) fetal bovine serum, 100 units/ml of penicillin, and 100 $\mu$g/ml of streptomycin.

Example 2-2: Analysis of gene expression in cells treated with peptide nucleic acid complexes

Cells of a mouse microglia cell line were seeded at $1 \times 10^5$ on a 6-well plate and cultured for 24 hours, the experiment was conducted under the conditions of Example 2-1, the cells were treated with 500 nM of the complex comprising the bioactive peptide nucleic acid and the carrier peptide nucleic acid and cultured for 24, 48, and 72 hours, and then 30 $\mu$L of RIPA buffer was added to each well to obtain protein lysate.

The protein lysate was assayed using a BCA assay kit (Thermo Fisher, USA) to quantitate the amount of protein, 30 $\mu$g of protein was separated by size through electrophoresis, and the protein was transferred to a PVDF membrane, treated at a ratio of 1:1000 with primary antibodies, namely, NLRP3 (abcam, USA), procaspase-1, caspase-1 (Santa Cruz Biotechnology, USA), proIL-1beta, and IL-1beta (Abcam, USA) and allowed to stand at 4°C for one day. The result was washed with 1X TBS-T, treated with secondary antibodies, namely, Goat Anti-Rabbit (Cell signaling Technology, USA) and Anti-Mouse (Santa Cruz Biotechnology, USA) at a ratio of 1:2000 and allowed to stand at room temperature for 1 hour. The result was treated with Supersignal™ West Femto Maximum Sensitivity Substrate (Thermo Fisher, USA) and the expression inhibition efficiency of the target gene was analyzed using Image600 (Amersham, Germany) equipment.

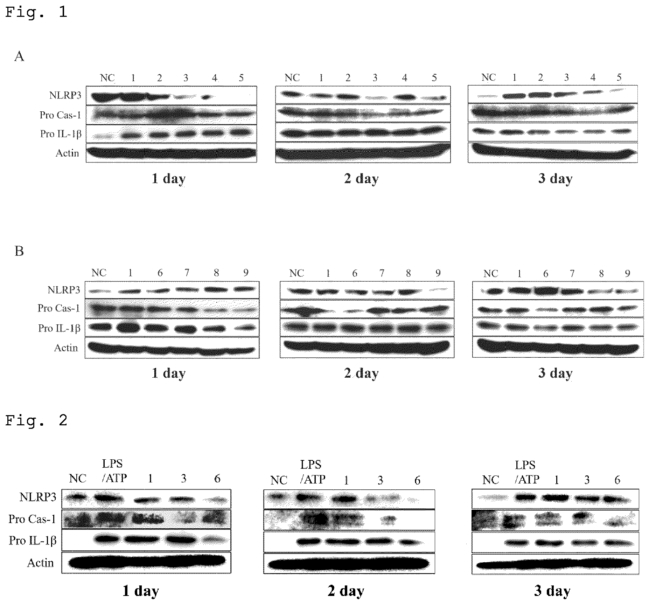

As a result, a group treated with a nucleic acid complex combination of SEQ ID NOS: 2 and 7 for SEQ ID NO: 2, as shown in FIG. 1A, and a group treated with a nucleic acid complex combination of SEQ ID NOS: 3 and 10 for SEQ ID NO: 3, as shown in FIG. 1B, most suppressed the expression of the target genes and the expression of the downstream genes over time.

## Example 3: Analysis of therapeutic effects on Alzheimer's dementia of nucleic acid complexes in primary cultured microglial cells isolated from brain tissue

In this example, the nucleic acid combinations whose effect was verified through Example 2 were selected and then the therapeutic effect for Alzheimer's disease was analyzed using primary microglia isolated from neonatal rats having the most similar culture state to the body.

Nucleic acid complex combinations that were used are shown in Table 3 below.

[Table 3]

| Nucleic acid complex combinations to determine therapeutic effect for Alzheimer's disease in primary microglia | |
| --- | --- |
| Combination | Nucleic acid complex |
| 1 | SEQ ID NOS: 4 and 5 |
| 3 | SEQ ID NOS: 2 and 7 |
| 6 | SEQ ID NOS: 3 and 10 |

Example 3-1: Isolation/culture of primary microglia from rat brain tissue and preparation of dementia-like inflammatory cell model

In order to isolate and culture primary microglia from brain tissues of rats, 1-day-old neonatal SD rats (Nara Biotech, Korea) were purchased and brain tissues were isolated therefrom. The meninges and other tissue parts were removed and the resulting cortex was transferred to a Petri dish containing iced 1x HBSS (Gibco, USA), and then the tissue was dissociated using a pipette and centrifuged at 1,200 rpm for 5 minutes. After centrifugation, the supernatant was discarded, the left pellet was added with a mixed glial cell culture medium supplemented with 10% (v/v) fetal bovine serum, 100 units/ml of penicillin, 100 ug/ml of streptomycin, and 0.1% GlutaMAX (Gibco, USA) to release the cells, and the cells were seeded in a 75T flask coated with PLL (poly-L-lysine, Sigma, USA) the day before. After 14 days of cell culture, the mixed glial cell culture medium was replaced with a microglia culture medium supplemented with 5 $\mu$g/ml of insulin (Sigma, USA), and then the flask was stirred with an orbital shaker for 2 hours at 160 rpm and 37°C. Then, the supernatant containing only floating cells was collected and centrifuged at 1,200 rpm for 8 minutes. After centrifugation, the cells were seeded at $1 \times 10^5$ in a 6-well plate, and cultured under conditions of 37°C and 5% (v/v) $CO_2$. In order to

construct an Alzheimer's dementia-like inflammatory cell model, 4 hours after treatment with the nucleic acid complex, the cells were further treated with LPS 100 ng/ml and ATP 5 mM, and then cultured.

Example 3-2: Analysis of gene expression in cells treated with peptide nucleic acid complexes

The experiment was conducted under the conditions of Example 3-1. 4 hours after treatment with 500 nM of the complex comprising the bioactive peptide nucleic acid and the carrier peptide nucleic acid, the cells were further treated with LPS 100 ng/ml and ATP 5 mM, and cultured for 24, 48, and 72 hours, and then 30 $\mu$L of RIPA buffer was added to each well to obtain a protein lysate.

The protein lysate was assayed using a BCA assay kit (Thermo Fisher, USA) to quantitate the amount of protein, 30 $\mu$g of protein was separated by size through electrophoresis, and the protein was transferred to a PVDF membrane, treated at a ratio of 1:1000 with primary antibodies, namely, NLRP3 (abcam, USA), procaspase-1, caspase-1 (Santa Cruz Biotechnology, USA), proIL-1beta, and IL-1beta (Abcam, USA) and allowed to stand at 4°C for one day. The result was washed with 1X TBS-T, secondary antibodies, namely, Goat Anti-Rabbit (Cell signaling Technology, USA) and Anti-Mouse (Santa Cruz Biotechnology, USA) at a ratio of 1:2000 and allowed to stand at room temperature for 1 hour. The result was treated with Supersignal™ West Femto Maximum Sensitivity Substrate (Thermo Fisher, USA) and the expression inhibition efficiency of the target gene was analyzed using Image600 (Amersham, Germany) equipment.

In this example, changes in expression of NLRP3 and downstream genes thereof were analyzed in an Alzheimer's dementia-like inflammatory cell model using LPS/ATP and the nucleic acid complex combinations used herein are shown in Table 3.

As a result, the nucleic acid complex combination (Combination 6) of SEQ ID NOS: 3 and 10, as shown in FIG. 2 most suppressed the expression of the target genes and the expression of the downstream genes over time.

## Example 4: Analysis of expression of Alzheimer's disease-causing genes in neuronal cells due to inflammatory response of primary microglia

In this example, changes in the expression of Alzheimer's disease-causing genes (tau and phosphorylation of tau) in neuronal cells that may occur due to the inflammatory response of microglia *in vivo,* were determined (Ising, C., Venegas, C., Zhang, S. et al., NLRP3 inflammasome activation drives tau pathology. Nature 575, 669-673 (2019)).

Example 4-1: Isolation/culture of microglia from brain tissue and preparation of dementia-like inflammatory cell model

Primary microglia were isolated from neonatal rats under the same conditions as in Example 3-1, and the cells were cultured under conditions of 37°C and 5% (v/v) $CO_2$. In order to construct an Alzheimer's dementia-like inflammatory cell model, 4 hours after treatment with the nucleic acid complex, the cells were further treated with LPS 100 ng/ml and ATP 5 mM for 3 or 6 hours, and then cultured.

Example 4-2: Isolation and culture of neuronal cells from brain tissue

Neonatal rats were purchased on the 7th day of microglia culture and neuron cells were isolated from brain tissue and cultured.

The hippocampus was separated from the isolated brain tissue using a stereomicroscope (Leica, USA), 0.25% Trypsin (Gibco, USA) and 10 $\mu$M DNase (Roche, Switzerland) were added thereto and the resulting mixture was allowed to stand at 37°C for 15 minutes. The tissue was homogenized using a pipette and then was centrifuged at 3,000 rpm for 5 minutes. The supernatant was discarded and the remaining pellet was added with an NM1 culture medium obtained by supplementing Neurobasal-A culture medium (Gibco, Korea) with 100 units/ml of penicillin, 100 ug/ml of streptomycin, and 2% B-27 supplement (Gibco, USA) to release the cells, and the cells for each group were seeded at $1 \times 10^5$ in a 6-well plate previously coated with PLL (poly-L-lysine, Sigma, USA) the day before. The isolated neuronal cells were added with NM1 culture medium supplemented with 5 $\mu$M Ara-c on DIV3 (days of in vitro) to prevent glial cells from growing, and it was observed under a microscope that only neuronal cells were cultured on the plate on DIV7.

Example 4-3: Microglia inflammatory activation and neuronal cell conditioning

In order to construct an Alzheimer's dementia-like inflammation model in microglia, the cells were seeded at $1 \times 10^5$ in a 6-well plate and treated with 100 ng/ml of LPS for 3 or 6 hours and with 5 mM ATP for 30 minutes after 24 hours and then cultured. The cells were washed once with 1xPBS (phosphate buffer saline, Welgene, Korea) and added with 2 ml of NM1 medium, which is a neuron cell culture medium, and then media conditioning was performed for 24 hours. After 24 hours of conditioning, the medium was filtered and B-27 supplement was added thereto, and the result was treated with neuronal cells again and cultured for 24 hours.

Example 4-5: Analysis of gene expression using Western blot assay

Microglia and neuron cells cultured under the experimental conditions of Example 4-4 were added with RIPA buffer at 30 μL/well to obtain a protein lysate. The protein lysate was assayed using a BCA assay kit (Thermo Fisher, USA) to quantitate the amount of protein, 30 μg of protein was separated by size through electrophoresis, and the protein was transferred to a PVDF membrane, treated at a ratio of 1:1000 with primary antibodies, namely, NLRP3 (abcam, USA), procaspase-1, caspase-1 (Santa Cruz Biotechnology, USA), proIL-1beta, IL-1beta (Abcam, USA), p-Tau (AT8) (Thermo Fisher, USA), and Tau (Thermo Fisher, USA) and allowed to stand at 4°C for one day. The result was washed with 1X TBS-T, treated with secondary antibodies, namely, Goat Anti-Rabbit (Cell signaling Technology, USA) and Anti-Mouse (Santa Cruz Biotechnology, USA) at a ratio of 1:2000 and allowed to stand at room temperature for 1 hour. The result was treated with Supersignal™ West Femto Maximum Sensitivity Substrate (Thermo Fisher, USA) and the expression inhibition efficiency of the target gene was analyzed in rat microglia using Image600 (Amersham, Germany) equipment.

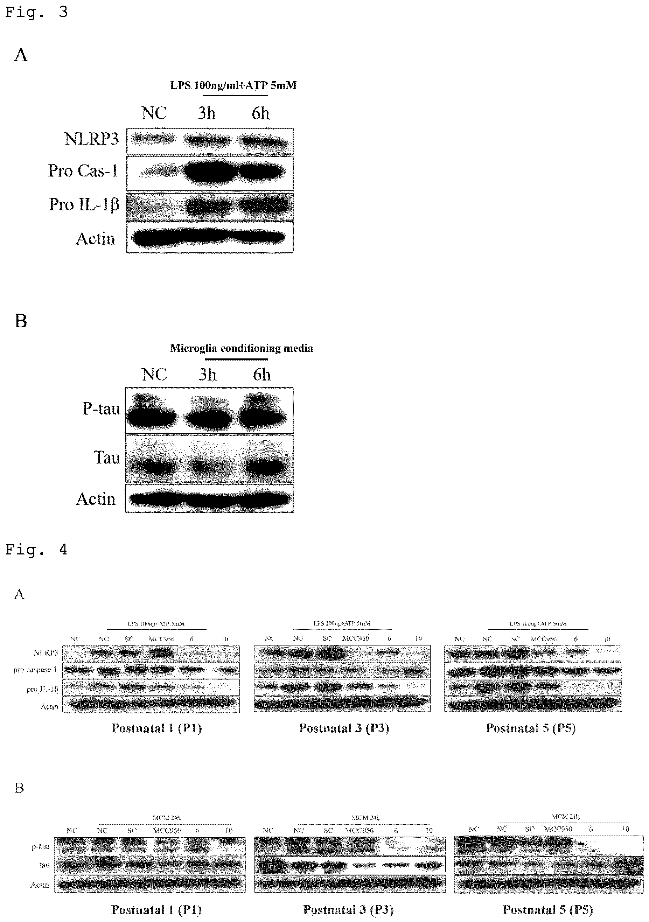

As a result, as can be seen from FIG. 3A, the target gene and downstream genes were overactivated in microglia the immune response of which was activated by treatment with LPS for 6 hours than for 3 hours, compared to the negative control group. In addition, as can be seen from FIG. 3B, the expression of phosphorylated tau (p-tau), the causative gene for Alzheimer's disease, increased over time in neuronal cells conditioned by activation of the immune response.

**Example 5: Analysis of therapeutic effects for Alzheimer's disease *in vitro* microglia and neuron cells using nucleic acid complexes capable of penetrating brain**

In this example, in order to confirm the expression of the causative gene for Alzheimer's disease in neuronal cells due to microglia inflammatory response in the brain using the nucleic acid complex, the effect of which was verified in Example 3 and the nucleic acid complex that had the identical sequence, but in which the peptide for facilitating endosomal escape was removed, *in vitro* experiments were performed using neonatal rats to determine whether or not to pass through the blood-brain and the effect of the nucleic acid complex on neuronal cells *in vivo.*

Nucleic acid complex combinations that are used are shown in Table 4 below.

[Table 4]

| Nucleic acid complex combinations to determine therapeutic effect for Alzheimer's disease in *in vitro* microglia and neuronal cells | |
| --- | --- |
| Item | Nucleic acid complex |
| 6 | SEQ ID NOS: 3 and 10 |
| 10 | SEQ ID NOS: 1 and 14 |

Example 5-1: Administration of complexes of bioactive peptide nucleic acid and carrier peptide nucleic acid to animal models

In order to determine whether or not the nucleic acid complex passes through the blood-brain barrier and the nucleic acid complex has an effect of inhibiting target genes *in vivo* microglia, SD rats (Nara Biotech, Korea) on the 16th day of pregnancy were purchased and then allowed to stand until the day of birth, and the nucleic acid complex prepared under the same conditions as in Example 5 was administered to the temporal vein of each young rat immediately after birth at 20 mg/kg. As a positive control, MCC950 (Invivogen, USA), which is an inhibitor of the NLRP3 gene, was used and administered intraperitoneally at 10 mg/kg. On days 1, 3, and 5 after injection, microglia were isolated from rats and then cultured.

Example 5-2: Isolation/culture of microglia from brain tissue and preparation of dementia inflammatory cell model

On the 1st, 3rd and 5th days after administration of the nucleic acid complex, microglia were isolated from rat brain tissue under the same conditions as in Example 3-1, and the cells were cultured at 37°C in the presence of 5% (v/v) $CO_2$. In order to construct an Alzheimer's dementia-like inflammatory cell model, the cells were treated with LPS 100 ng/ml for 6 hours and ATP 5 mM for 30 minutes and then cultured.

Example 5-3: Isolation and culture of neuronal cells from brain tissue

Under the same conditions as in Example 4-2, neuron cells were isolated from brain tissue of neonatal rats and cultured. It was observed under a microscope that only neuronal cells were cultured on the plate on DIV7.

Example 5-4: Microglia inflammatory activation and neuronal cell conditioning

In order to construct an Alzheimer's dementia-like inflammation model in microglia, the cells were seeded at $1\times10^5$ in a 6-well plate and treated with 100 ng/ml of LPS for 6 hours and 5 mM ATP for 30 minutes after 24 hours and then cultured. The cells were washed once with 1x PBS (phosphate buffer saline, Welgene, Korea) and added with 2 ml of NM1 medium, which is a neuron cell culture medium, and then media conditioning was performed for 24 hours. After 24 hours of conditioning, the medium was filtered, B-27 supplement was added thereto, and the result was treated with neuronal cells again and cultured for 24 hours.

Example 5-5: Analysis of gene expression using Western blot assay

Microglial and neuron cells cultured under the experimental conditions of Example 5-4 were added with RIPA buffer at 30 μL/well to obtain a protein lysate. The protein lysate was assayed using a BCA assay kit (Thermo Fisher, USA) to quantitate the amount of protein, 30 μg of protein was separated by size through electrophoresis, and the protein was transferred to a PVDF membrane, treated at a ratio of 1:1000 with primary antibodies, namely, NLRP3 (abcam, USA), procaspase-1, caspase-1 (Santa Cruz Biotechnology, USA), proIL-1beta, IL-1beta (Abcam, USA), p-Tau (AT8) (Thermo Fisher, USA), and Tau (Thermo Fisher, USA) and allowed to stand at 4°C for one day. The result was washed with 1X TBS-T, treated with secondary antibodies, namely, Goat Anti-Rabbit (Cell signaling Technology, USA) and Anti-Mouse (Santa Cruz Biotechnology, USA) at a ratio of 1:2000 and allowed to stand at room temperature for 1 hour. The result was treated with Supersignal™ West Femto Maximum Sensitivity Substrate (Thermo Fisher, USA) and the expression inhibition efficiency of the target gene was analyzed in rat microglia using Image600 (Amersham, Germany) equipment.

As a result, as can be seen from FIG. 4A, despite the activation of the immune response in microglia due to the treatment with LPS and ATP, the groups administered the nucleic acid complex combination of SEQ ID NOS: 3 and 10 (combination 6) and the nucleic acid complex combination of SEQ ID NOS: 1 and 14 (combination 10) exhibited a decrease in the expression of the target gene and downstream gene over time, compared to the immune response induction control and the positive control (MCC950: NLRP3 inhibitor).

In addition, changes in Alzheimer's dementia-related genes in neuronal cells due to overactivation of the immune response of microglia were observed. As can be seen from FIG. 4B, the result shows that the neuron cells in the medium conditioned in microglia in which the expression of the target gene is inhibited by the nucleic acid complex of SEQ ID NOS: 3 and 10 (Combination 6) and the nucleic acid complex of SEQ ID NOS: 1 and 14 (Combination 10) exhibited a decrease in the expression of the phosphorylated tau (p-tau) protein, the causative gene for Alzheimer's disease, over time, compared to other controls.

In particular, it was confirmed that the effect of inhibiting the expressions of the target gene (NLRP3) and the downstream gene thereof and Alzheimer's disease causative gene (p-tau) in microglial and neuron cells occur rapidly by the nucleic acid complex without the peptide for facilitating endosomal escape (SEQ ID NOS: 1 and 14, combination 10), compared to the nucleic acid complex combination (SEQ ID NOS: 3 and 10, combination 6) with the peptide for facilitating endosomal escape (FIGS. 4A and 4B).

**Industrial Applicability**

The nucleic acid complex according to the present invention is capable of penetrating the blood-brain barrier and efficiently inhibiting protein expression and phosphorylation of the NLRP3 gene and the downstream gene thereof, i.e. tau, and thus is useful for prevention or treatment of dementia.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

**Sequence List Free Text**

An electronic file is attached.

**Claims**

1. A pharmaceutical composition for preventing, ameliorating or treating Alzheimer's dementia comprising a cell-permeable nucleic acid complex in which a bioactive nucleic acid targeting an NLRP3 gene and a carrier peptide nucleic acid complementarily bound to each other, as an active ingredient.

2. The pharmaceutical composition according to claim 1, wherein the bioactive peptide nucleic acid comprises sequences represented by SEQ ID NOS: 1 to 3.

3. The pharmaceutical composition according to claim 1, wherein the carrier peptide nucleic acid comprises a sequence selected from the group consisting of sequences represented by SEQ ID NOS: 5 to 14.

4. The pharmaceutical composition according to claim 1, wherein the nucleic acid complex is selected from the group consisting of the following nucleic acid complexes:

   (i) a nucleic acid complex comprising a bioactive nucleic acid having a sequence represented by SEQ ID NO: 1 and a carrier peptide nucleic acid having a sequence represented by SEQ ID NO: 14;
   (ii) a nucleic acid complex comprising a bioactive nucleic acid having a sequence represented by SEQ ID NO: 3 and a carrier peptide nucleic acid having a sequence represented by SEQ ID NO: 10; and
   (iii) a nucleic acid complex comprising a bioactive nucleic acid having a sequence represented by SEQ ID NO: 2 and a carrier peptide nucleic acid having a sequence represented by SEQ ID NO: 7.

5. The pharmaceutical composition according to claim 1, wherein the bioactive nucleic acid or the carrier peptide nucleic acid is further bound with a substance for facilitating endosomal escape to a 5'-end or 3'-end of each nucleic acid.

6. The pharmaceutical composition according to claim 5, wherein the substance for facilitating endosomal escape is at least one selected from the group consisting of peptides, lipid nanoparticles, polyplex nanoparticles, polymer nanospheres, inorganic nanoparticles, cationic lipid-based nanoparticles, cationic polymers, and pH-sensitive polymers.

7. The pharmaceutical composition according to claim 6, wherein the peptide that facilitates endosomal escape is GLFDIIKKIAESF (SEQ ID NO: 15) or histidine (10).

8. The pharmaceutical composition according to claim 1, wherein the bioactive nucleic acid has an overall negative charge or neutral.

9. The pharmaceutical composition according to claim 1, wherein the carrier peptide nucleic acid has an overall positive charge.

10. The pharmaceutical composition according to claim 1, wherein the nucleic acid complex has an overall positive charge.

11. The pharmaceutical composition according to claim 1, wherein the nucleic acid complex is capable of penetrating a blood-brain barrier.

12. The pharmaceutical composition according to claim 1, wherein the dementia is selected from the group consisting of Alzheimer's disease, vascular dementia, dementia caused by Parkinson's disease, Lewy body dementia, dementia caused by Huntington's disease, dementia caused by Creutzfeldt-Jacob disease, and dementia caused by Pick's disease.

Fig. 1

A

**1 day**          **2 day**          **3 day**

B

**1 day**          **2 day**          **3 day**

Fig. 2

**1 day**          **2 day**          **3 day**

Fig. 3

A

B

Fig. 4

A

B

## INTERNATIONAL SEARCH REPORT

| | International application No. |
|---|---|
| | **PCT/KR2021/013049** |

### A. CLASSIFICATION OF SUBJECT MATTER

**A61K 48/00**(2006.01)i; **A61K 31/7088**(2006.01)i; **A61K 47/54**(2017.01)i; **A61K 47/64**(2017.01)i; **A61P 25/28**(2006.01)i

According to International Patent Classification (IPC) or to both national classification and IPC

### B. FIELDS SEARCHED

Minimum documentation searched (classification system followed by classification symbols)

A61K 48/00(2006.01); A61K 31/7105(2006.01); A61K 45/00(2006.01); C12N 15/09(2006.01); C12Q 1/68(2006.01); C12Q 1/6883(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: NLRP3(NLR Family Pyrin Domain Containing 3), 생활성 핵산(bioactive nucleic acid), 캐리어 펩티드 핵산(carrier peptide nucleic acid), 세포 투과성(cell permeability), 알츠하이머(alzheimer), 치매 (dementia)

### C. DOCUMENTS CONSIDERED TO BE RELEVANT

| Category* | Citation of document, with indication, where appropriate, of the relevant passages | Relevant to claim No. |
|---|---|---|
| Y | JP 2018-537528 A (DERA PHARMACEUTICALS INC.) 20 December 2018 (2018-12-20)<br>See paragraphs [0055], [0095] and [0113] and claims 1 and 24. | 1,5-12 |
| A | | 2-4 |
| Y | KR 10-2019-0096148 A (SEASUN THERAPEUTICS) 19 August 2019 (2019-08-19)<br>See paragraphs [0038], [0115] and [0134], claims 1-22 and figures 1a-1e. | 1,5-12 |
| A | PAL, A. et al. Inhibition of NLRP3 inflammasome activation by cell-permeable stapled peptides. Scientific Reportes. 2019, vol. 9, 4913, inner pp. 1-15(publication date: 20 March 2019).<br>See abstract. | 1-12 |
| A | KR 10-2018-0018405 A (SEASUN BIOMATERIALS) 21 February 2018 (2018-02-21)<br>See entire document. | 1-12 |

☑ Further documents are listed in the continuation of Box C.       ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 December 2021** | **28 December 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| | International application No. |
|---|---|
| | **PCT/KR2021/013049** |

**C.    DOCUMENTS CONSIDERED TO BE RELEVANT**

| Category* | Citation of document, with indication, where appropriate, of the relevant passages | Relevant to claim No. |
|---|---|---|
| A | JP 2008-220366 A (NATIONAL INSTITUTE OF ADVANCED INDUSTRIAL & TECHNOLOGY et al.) 25 September 2008 (2008-09-25) See entire document. | 1-12 |

Form PCT/ISA/210 (second sheet) (July 2019)

**INTERNATIONAL SEARCH REPORT**

International application No.

**PCT/KR2021/013049**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed:

          ☑  in the form of an Annex C/ST.25 text file.

          ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐  in the form of an Annex C/ST.25 text file (Rule 13*ter*.1(a)).

          ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

INTERNATIONAL SEARCH REPORT
Information on patent family members

International application No.

**PCT/KR2021/013049**

| Patent document cited in search report | | | Publication date (day/month/year) | Patent family member(s) | | | Publication date (day/month/year) |
|---|---|---|---|---|---|---|---|
| JP | 2018-537528 | A | 20 December 2018 | CN | 109196118 | A | 11 January 2019 |
| | | | | EP | 3371328 | A2 | 12 September 2018 |
| | | | | US | 2017-145412 | A1 | 25 May 2017 |
| | | | | WO | 2017-079352 | A2 | 11 May 2017 |
| KR | 10-2019-0096148 | A | 19 August 2019 | AU | 2019-219472 | A1 | 15 August 2019 |
| | | | | AU | 2019-219472 | A1 | 10 September 2020 |
| | | | | CA | 3090754 | A1 | 15 August 2019 |
| | | | | CN | 112135907 | A | 25 December 2020 |
| | | | | EP | 3750995 | A1 | 16 December 2020 |
| | | | | JP | 2021-515589 | A | 24 June 2021 |
| | | | | KR | 10-2262260 | B1 | 09 June 2021 |
| | | | | US | 2021-0171953 | A1 | 10 June 2021 |
| | | | | WO | 2019-156365 | A1 | 15 August 2019 |
| KR | 10-2018-0018405 | A | 21 February 2018 | AU | 2017-310146 | A1 | 28 March 2019 |
| | | | | AU | 2017-310146 | A1 | 15 February 2018 |
| | | | | AU | 2017-310146 | B2 | 17 December 2020 |
| | | | | CA | 3033474 | A1 | 15 February 2018 |
| | | | | CN | 109804070 | A | 24 May 2019 |
| | | | | EP | 3498844 | A1 | 19 June 2019 |
| | | | | JP | 2019-526624 | A | 19 September 2019 |
| | | | | JP | 6818889 | B2 | 20 January 2021 |
| | | | | KR | 10-1963885 | B1 | 01 April 2019 |
| | | | | KR | 10-2018-0100523 | A | 11 September 2018 |
| | | | | US | 10745446 | B2 | 18 August 2020 |
| | | | | US | 2019-0185519 | A1 | 20 June 2019 |
| | | | | WO | 2018-030789 | A1 | 15 February 2018 |
| JP | 2008-220366 | A | 25 September 2008 | | None | | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2017008636 W
- KR 1020190128465

**Non-patent literature cited in the description**

- **SELKOE.** *Physiol Rev,* 2001, vol. 81, 741-66
- **ISING, C et al.** *Nature,* 2019, vol. 575, 669-673
- **KOLE R et al.** *Nature Rev. Drug Discov,* 2012, vol. 11, 125
- **WILSON C et al.** *Curr. Opin. Chem. Bio,* 2006, vol. 10 (607), 2006
- **D. W. PACK ; A. S. HOFFMAN ; S. PUN ; P. S. STAYTON.** Design and development of polymers for gene delivery. *Nat. Rev. Drug. Discov.,* 2005, vol. 4, 581-593
- **ISING, C. ; VENEGAS, C. ; ZHANG, S et al.** NLRP3 inflammasome activation drives tau pathology. *Nature,* 2019, vol. 575, 669-673